# EUROPEAN PATENT SPECIFICATION

(11) **EP 4 680 710 B1**
(45) Date of publication and mention of the grant of the patent: **09.09.2026**
(21) Application number: 24711216.2
(22) Date of filing: 15.03.2024
(51) Int. Cl.: C11D 3/386, C11D 11/00, C12N 9/00, C12N 9/20, C12N 9/96

(54) **COMPOSITION**
ZUSAMMENSETZUNG
COMPOSITION

(30) Priority: 17.03.2023 EP 23162591
(43) Date of publication of application: 21.01.2026
(73) Proprietor: Unilever IP Holdings B.V., 6708 WH Wageningen (NL); Unilever Global IP Limited, Wirral, Merseyside CH62 4ZD (GB)
(72) Inventor: LITTLECHILD-BOND, Jennifer Ann, 6708 WH Wageningen (NL); O'HALLORAN, Samuel Ellis, 6708 WH Wageningen (NL); PARRY, Neil James, 6708 WH Wageningen (NL); RENDERS, Marleen, 6708 WH Wageningen (NL)
(74) Representative: Unilever Patent Group
(86) International application number: PCT/EP2024/056995
(87) International publication number: WO 2024/194190

(56) References cited:
- WO-A1-2013/024143
- WO-A1-2017/036901
- WO-A1-2022/136389
- WO-A1-97/24428
- US-A1- 2021 130 865
- MAIANGWA JONATHAN ET AL: "Adaptational properties and applications of cold-active lipases from psychrophilic bacteria", EXTREMOPHILES, SPRINGER JAPAN, TOKYO, vol. 19, no. 2, 4 December 2014 (2014-12-04), pages 235 - 247, XP035456275, ISSN: 1431-0651, [retrieved on 20141204], DOI: 10.1007/S00792-014-0710-5
- KLEMETSEN T: "Moritella viscosa Lipase family protein", INTERNATIONAL NUCLEOTIDE SEQUENCE DATABASE COLLABORATION, 21 November 2016 (2016-11-21), pages 1 - 2, XP093076657, Retrieved from the Internet <URL:https://www.ebi.ac.uk/ena/browser/api/embl/SGZ09220.1?download=true> [retrieved on 20230828]

## Description

### Field of Invention

The invention concerns a composition, in particular a detergent composition, comprising a particular lipase enzyme.

### Background of the Invention

A useful ingredient that in cleaning compositions, particularly detergent compositions is the incorporation of lipase enzymes. These work particularly well cleaning fatty stains.

WO 2017/036901 A1 discloses laundry compositions comprising lipases from *Psychromonas ingrahamii* and uses of such compositions in methods of laundry, especially at low temperature.

WO 2013/024143 A1 discloses an enzymatic fabric treatment composition comprising one or more psychrophilic enzymes. WO 97/24428 A1 discloses detergent compositions containing an enzyme produced by a psychrophilic or psychrotrophic organism and having an optimum activity between 5 and 40°C.

However, lipases are expensive ingredients and there is always a need to improve their efficacy.

It is an object of the invention to improve the efficacy of lipase against fatty stains, particularly margarine.

It is also an object of the invention to improve the stability of lipase in combination with other general detergent ingredients, particularly proteases and soil release polymers.

### Summary of the Invention

We have found that a lipase from *Moritella viscosa* solves these problems.

In one aspect the invention provides a detergent composition comprising:
(a) from 0.0005 to 6 wt.%, preferably from 0.005 to 4 wt.%, more preferably from 0.001 to 2 wt.% wt.% of a lipase from *Moritella viscosa*; and,
(b) from 1 to 60 wt.% preferably from 1 to 50 wt.%, more preferably from 1 to 35 wt.% of a detersive surfactant, wherein the lipase from *Moritella viscosa* has a sequence identity of at least 70% with SEQ. ID. 1.

Preferably the invention provides a detergent composition comprising:
(a) from 0.0005 to 6 wt.%, preferably from 0.005 to 4 wt.%, more preferably from 0.001 to 2 wt.% wt.% of a lipase from *Moritella viscosa*; and,
(b) from 1 to 60 wt.% preferably from 2 to 50 wt.%, more preferably from 4 to 35 wt.% of a detersive surfactant, wherein the lipase from Moritella viscosa has a sequence identity of at least 70% with SEQ. ID. 1.

Preferably the lipase from *Moritella viscosa* has a sequence identity of at least 75%, preferably 80%, more preferably 85%, even more preferably 90%, even more preferably 95%, even more preferably 98%, even more preferably 99%, most preferably 100% with SEQ. ID. 1.

Preferably the anionic surfactant is present at a level of from 1 to 50 wt.%, preferably from 2 to 40 wt.%, more preferably from 3 to 30 wt.% and is preferably selected from linear alkyl benzenesulphonate, secondary alkane sulphonate, sodium laureth ether sulphate, sodium lauryl sulphate, sodium oleyl sulphate and sodium oleyl ether sulphate, methyl ester sulphonate, secondary alkyl sulphate (SALS), cardanol ether sulphate and a rhamnolipid. Preferably the nonionic surfactant is present at a level of from 1 to 30 wt.%, preferably from 2 to 20 wt.%, more preferably from 3 to 15 wt.% and is preferably selected from an alcohol ethoxylate, an alcohol propoxylate, a methyl ester ethoxylate and an alkyl poly glycoside.

A preferred detergent composition is a laundry detergent composition. Preferably the laundry detergent composition is in the form of a liquid, solid, powder, pastille, bead or paste. More preferably the composition is a liquid or a powder, most preferably a liquid detergent. The laundry detergent preferably comprises an alkoxylated polyamine, preferably at a level of from 0.1 to 8 wt.%, more preferably from 0.2 to 6 wt.%, most preferably from 0.5 to 5 wt.%.

The laundry detergent preferably comprises soil release polymer, the soil release polymer preferably selected from copolyesters of dicarboxylic acids and polydiols, more preferably a copolyester formed by condensation of terephthalic acid ester and 1,2-propanediol, the soil release polymer preferably present at a level of from 0.1 to 8 wt.%, more preferably from 0.2 to 6 wt.%, most preferably from 0.5 to 5 wt.%.

Preferred detergent compositions, particularly laundry detergent compositions additionally comprises one or more further enzymes selected from the group consisting of: proteases, cellulases, alpha-amylases, peroxidases/oxidases, pectate lyases, and/or mannanases. Preferably the one or more enzymes comprises protease.

In another aspect the invention provides a method of treatment of a textile having a fatty stain, wherein said textile is treated with a detergent composition according the first aspect of the invention, to provide enhanced lipolytic cleaning against fatty stains, preferably margarine stains, said textile preferably subsequently rinsed and dried.

In another aspect the invention provides a method of treatment of a textile having a fatty stain, wherein said textile is pre-treated with a composition according to the first aspect of the invention, to provide enhanced lipolytic cleaning against fatty stains, preferably margarine stains, said textile then subsequently washed with a laundry main wash composition, and then preferably subsequently rinsed and dried.

In another aspect the present invention provides the use of a lipase from *Moritella viscosa* having a sequence identity of at least 70% with SEQ. ID. 1, to improve cleaning of fatty stains, preferably margarine stains on textiles. More preferably the present invention provides the use of a detergent composition comprising a lipase from *Moritella viscosa* having a sequence identity of at least 70% with SEQ. ID. 1, to improve cleaning of fatty stains, preferably margarine stains on textiles.

### Detailed Description of the Invention

The indefinite article "a" or "an" and its corresponding definite article "the" as used herein means at least one, or one or more, unless specified otherwise.

All % levels of ingredients in compositions (formulations) listed herein are in wt.% based on total formulation unless other stated.

It is understood that any reference to a preferred ingredient of the detergent composition is envisaged to be combinable subject matter with any other preferred ingredient of the detergent composition disclosed herein.

The detergent composition can be applied to any suitable substrate. Particularly preferred substrates are textiles. Particularly preferred detergent compositions are laundry detergent compositions.

Laundry detergent compositions may take any suitable form. Preferably the laundry detergent composition is in the form of a liquid, solid, powder, pastille, bead or paste, preferably the composition is a liquid or a powder, more preferably a liquid detergent.

### Lipases from Moritella viscosa

Lipases (E.C. 3.1.1.3) are hydrolytic enzymes that are known to cleave ester bonds in lipids. The lipase of the invention is from the species *Moritella viscosa.* These lipases from *Moritella viscosa* are termed herein as MorvLip.

The lipase from *Moritella viscosa* has a sequence identity of at least 70% with SEQ. ID. 1. Preferably the lipase from *Moritella viscosa* has a sequence identity of at least 75%, preferably 80%, more preferably 85%, even more preferably 90%, even more preferably 95%, even more preferably 98%, even more preferably 99%, most preferably 100% with SEQ. ID. 1.

The composition comprises from 0.0005 to 6 wt.%, preferably from 0.005 to 4 wt.%, more preferably from 0.001 to 2 wt.% of a lipase from *Moritella viscosa.* Other preferred amounts include from 0.001 to 1 wt.% of said lipase.

### Percentage Sequence Identity

Percentage (%) sequence identity is defined as the percentage of amino acid residues in a candidate sequence that are identical with residues in the given listed sequence (referred to by the SEQ ID No.) after aligning the sequences and introducing gaps as necessary, to achieve the maximum sequence identity, and not considering any conservative substitutions as part of the sequence identity. Sequence identity is calculated over the entire length of the respective sequences.

Where the aligned sequences are of different length, sequence identity of the shorter comparison sequence may be determined over the entire length of the longer given sequence or, where the comparison sequence is longer than the given sequence, sequence identity of the comparison sequence may be determined over the entire length of the shorter given sequence.

For example, where a given sequence comprises 100 amino acids and the candidate sequence comprises 10 amino acids, the candidate sequence can only have a maximum identity of 10% to the entire length of the given sequence. This is further illustrated in the following example:
(A)

| | | |
|---|---|---|
| Given seq: | XXXXXXXXXXXXXXX | (15 amino acids) |
| Comparison seq: | XXXXXYYYYYYY | (12 amino acids) |

% sequence identity = the number of identically matching amino acid residues after alignment divided by the total number of amino acid residues in the longer given sequence, i.e. (5 divided by 15) x 100 = 33.3%

Where the comparison sequence is longer than the given sequence, sequence identity may be determined over the entire length of the given sequence. For example:
(B)

| | | |
|---|---|---|
| Given seq: | XXXXXXXXXX | (10 amino acids) |
| Comparison seq: | XXXXXYYYYYYZZYZZZZZZ | (20 amino acids) |

% sequence identity = number of identical amino acids after alignment divided by total number of amino acid residues in the given sequence, i.e. (5 divided by 10) x 100 = 50%.

Alignment for purposes of determining percent amino acid sequence identity can be achieved in various ways known to a person of skill in the art, for instance, using publicly available computer software such as ClustalW 1.82. T-coffee or Megalign (DNASTAR) software. When using such software, the default parameters, e.g. for gap penalty and extension penalty, are preferably used. The default parameters of ClustalW 1.82 are: Protein Gap Open Penalty = 10.0, Protein Gap Extension Penalty = 0.2, Protein matrix = Gonnet, Protein/DNA ENDGAP = -1, Protein/DNA GAPDIST = 4.

Identity of nucleic acid sequences may be determined in a similar manner involving aligning the sequences and introducing gaps if necessary, to achieve the maximum sequence identity, and calculating sequence identity over the entire length of the respective sequences. Where the aligned sequences are of different length, sequence identity may be determined as described above and illustrated in examples (A) and (B).

The most preferred lipase is given by Sequence ID No. 1 (SEQ. ID. 1). Letters refer to amino acids of the protein sequence.

SEQ. ID. 1
MHKKIISPTDSLKTQHLELLESQVPHYRQAYSDRTAWLMACMSELAYVKFNPLYPN
KFTEEAISSTLQKLSSTTLDPRVGKILSTLSSMSYDHVEEKKLLESELSELNGAILLDT
FDCNGTQAMLVELASFNILAFRGTEATSLKDIKADIKAVTTRCETGGQVHSGFKDAF
DEVCDSIVGALKAINNNKPLMITGHSLGGALATIAAKRLTFKNGIAGCYTFGSPRVGD
DKWISTVKTPIYRVVNAADVVTMLPPNSIAIESASAVFSLVPYAGEVIKNTLLSKFNG
YIHGGNMRYLTNCEAGNYANVKLLYSVSFLYRIKALLMGKLPVKKLAADHSISIYRQ
KLALVALQRKSLGDQTIKTGTKQ

### Surfactant

The composition is a detergent composition. The detergent composition comprises surfactant (which may include a mixture of two or more surfactants). The composition comprises from 1 to 60 wt.%, more preferably from 1 to 50 wt.%, most preferably from 1 to 35 wt.% of a detersive surfactant.

More preferably the composition comprises from 1 to 60 wt.%, more preferably from 2 to 50 wt.%, most preferably from 4 to 35 wt.% of a detersive surfactant. Even more preferred levels of surfactant are from 6 to 35 wt.%, more preferably from 8 to 35 wt.%.

The detergent composition (preferably a laundry detergent composition) comprises anionic and/or nonionic surfactant, preferably comprising both anionic and nonionic surfactant.

Anionic Surfactant are described in Anionic Surfactants Organic Chemistry (Surfactant Science Series Volume 56) edited By H.W.Stache (Marcel Dekker 1996).

Preferably, the composition comprises from 1 to 50 wt.%, preferably from 2 to 40 wt.%, more preferably from 3 to 30 wt.% anionic surfactant based on the total weight of composition. Non-soap anionic surfactants for use in the invention are typically salts of organic sulphates and sulphonates having alkyl radicals containing from about 8 to about 22 carbon atoms, the term "alkyl" being used to include the alkyl portion of higher acyl radicals. Examples of such materials include alkyl sulphates, alkyl ether sulphates, alkaryl sulfonates, alphaolefin sulfonates and mixtures thereof. The alkyl radicals preferably contain from 10 to 18 carbon atoms and may be unsaturated. The alkyl ether sulphates may contain from one to ten ethylene oxide or propylene oxide units per molecule, and preferably contain one to three ethylene oxide units per molecule. The counterion for anionic surfactants is generally an alkali metal such as sodium or potassium; or an ammoniacal counterion such as monoethanolamine, (MEA) diethanolamine (DEA) or triethanolamine (TEA). Mixtures of such counterions may also be employed.

The compositions according to the invention may include alkylbenzene sulfonates, particularly linear alkylbenzene sulfonates (LAS) with an alkyl chain length of from 10 to 18 carbon atoms. Commercial LAS is a mixture of closely related isomers and homologues alkyl chain homologues, each containing an aromatic ring sulphonated at the "*para*" position and attached to a linear alkyl chain at any position except the terminal carbons. The linear alkyl chain typically has a chain length of from 11 to 15 carbon atoms, with the predominant materials having a chain length of about C12. Each alkyl chain homologue consists of a mixture of all the possible sulphophenyl isomers except for the 1 -phenyl isomer. LAS is normally formulated into compositions in acid (i.e. HLAS) form and then at least partially neutralized in-situ.

Some alkyl sulphate surfactant (PAS) may be used, such as non-ethoxylated primary and secondary alkyl sulphates with an alkyl chain length of from 10 to 18.

Also commonly used in laundry liquid compositions are alkyl ether sulfates having a straight or branched chain alkyl group having 10 to 18, more preferably 12 to 14 carbon 30 atoms and containing an average of 1 to 3EO units per molecule. A preferred example is sodium lauryl ether sulfate (SLES) in which the predominantly C12 lauryl alkyl group has been ethoxylated with an average of 3EO units per molecule.

The alkyl ether sulphate may be provided in a single raw material component or by way of a mixture of components.

Preferred anionic surfactants also include the C16/18 alkyl ether sulphates.

Preferred anionic surfactants also include rhamnolipids.

The anionic surfactant is preferably selected from linear alkyl benzenesulphonate, secondary alkane sulphonate, sodium laureth ether sulphate, sodium lauryl sulphate, sodium oleyl sulphate and sodium oleyl ether sulphate, methyl ester sulphonate, secondary alkyl sulphate (SALS), cardanol ether sulphate and a rhamnolipid.

Mixtures of any of the above described materials may also be used.

Preferably the detergent composition comprises non-ionic surfactant, preferably from 1 to 30 wt.%, preferably from 2 to 20 wt.%, more preferably from 3 to 15 wt.% of non-ionic surfactant.

Suitable nonionic detergent compounds which may be used include, in particular, the reaction products of compounds having an aliphatic hydrophobic group and a reactive hydrogen atom, for example, aliphatic alcohols, acids or amides, especially ethylene oxide either alone or with propylene oxide. The nonionic surfactant is preferably selected from an alcohol ethoxylate, an alcohol propoxylate, a methyl ester ethoxylate and an alkyl poly glycoside.

Preferred nonionic detergent compounds are the condensation products of aliphatic C₈ to C₁₈ primary or secondary linear or branched alcohols with ethylene oxide.

Most preferably the nonionic detergent compound is the alkyl ethoxylated non-ionic surfactant is a C₈ to C₁₈ primary alcohol with an average ethoxylation of 7EO to 9EO units.

Preferably the surfactants used are saturated.

A composition of the invention may contain one or more amphoteric (such as zwitterionic surfactants), preferably wherein if present, the amphoteric surfactant is present at a level of from 0.1 to 15 wt.%, preferably from 0.5 to 10 wt.%, more preferably from 1 to 5 wt.% and is preferably selected from alkyl betaines and the alkyl sulphobetaines (sultaines), more preferably carbobetaines and lauramine oxide.

Further specific amphoteric surfactants include alkyl amine oxides, alkyl amidopropyl betaines, alkyl glycinates, alkyl carboxyglycinates, alkyl amphoacetates, alkyl amphopropionates, alkylamphoglycinates, alkyl amidopropyl hydroxysultaines, acyl taurates and acyl glutamates.

The amphoteric surfactant preferably comprises alkyl radicals containing from about 8 to about 22 carbon atoms preferably selected from C12, C14, C16, C18 and C18:1, the term "alkyl" being used to include the alkyl portion of higher acyl radicals.

Mixtures of any of the above described materials may also be used.

### Soil release polymer

When the detergent composition is in the form of a laundry composition, it is preferred that a soil release polymer is included.

The soil release polymer is preferably present at a level of from 0.1 to 10 wt.%.

The levels of soil release polymer are preferably from 0.1 to 8 wt.%, more preferably from 0.2 to 6 wt.%, most preferably from 0.5 to 5 wt.%.

Preferably the soil release polymer is a polyester based soil released polymer. More preferably the polyester soil release polymer is selected from copolyesters of dicarboxylic acids and polydiols. More preferably the soil release polymer is a polyethylene and/or polypropylene terephthalate based soil release polymer, most preferably a polypropylene terephthalate based soil release polymer, most preferably a copolyester formed by condensation of terephthalic acid ester and 1,2-propanediol.

Suitable polyester based soil release polymers are described in WO 2014/029479 and WO 2016/005338.

### Alkoxylated polyamine

When the detergent composition is in the form of a laundry composition, it is preferred that an alkoxylated polyamine is included.

Preferred levels of alkoxylated polyamine range from 0.1 to 8 wt.%, preferably from 0.2 to 6 wt.%, more preferably from 0.5 to 5 wt.%. Another preferred level is from 1 to 4 wt.%.

The alkoxylated polyamine may be linear or branched. It may be branched to the extent that it is a dendrimer. The alkoxylation may typically be ethoxylation or propoxylation, or a mixture of both. Where a nitrogen atom is alkoxylated, a preferred average degree of alkoxylation is from 10 to 30, preferably from 15 to 25.

A preferred material is alkoxylated polyethylenimine, most preferably ethoxylated polyethyleneimine, with an average degree of ethoxylation being from 10 to 30 preferably from 15 to 25, where a nitrogen atom is ethoxylated.

### Additional Enzymes

Additional enzymes, other than the specified lipase may be present in the detergent composition. It is preferred that additional enzymes are present in the preferred laundry detergent composition.

If present, then the level of each additional enzyme in the laundry composition of the invention is from 0.0001 wt.% to 0.1 wt.%.

Levels of enzyme present in the composition preferably relate to the level of enzyme as pure protein.

Preferred further enzymes include those in the group consisting of: proteases, cellulases, alpha-amylases, peroxidases/oxidases, pectate lyases, and/or mannanases. Said preferred additional enzymes include a mixture of two or more of these enzymes.

Preferably the further enzyme is selected from: proteases, cellulases, and/or alpha-amylases. Most preferably the additional enzyme comprises protease.

Protease enzymes hydrolyse bonds within peptides and proteins, in the laundry context this leads to enhanced removal of protein or peptide containing stains. Examples of suitable proteases families include aspartic proteases; cysteine proteases; glutamic proteases; aspargine peptide lyase; serine proteases and threonine proteases. Such protease families are described in the MEROPS peptidase database (http://merops.sanger.ac.uk/). Serine proteases are preferred. Subtilase type serine proteases are more preferred. The term "subtilases" refers to a sub-group of serine protease according to Siezen et al., Protein Engng. 4 (1991) 719-737 and Siezen et al. Protein Science 6 (1997) 501 -523. Serine proteases are a subgroup of proteases characterized by having a serine in the active site, which forms a covalent adduct with the substrate. The subtilases may be divided into 6 subdivisions, i.e. the Subtilisin family, the Thermitase family, the Proteinase K family, the Lantibiotic peptidase family, the Kexin family and the Pyrolysin family.

Examples of subtilases are those derived from Bacillus such as Bacillus lentus, B. alkalophilus, B. subtilis, B. amyloliquefaciens, Bacillus pumilus and Bacillus gibsonii described in; US7262042 and WO09/021867, and subtilisin lentus, subtilisin Novo, subtilisin Carlsberg, Bacillus licheniformis, subtilisin BPN', subtilisin 309, subtilisin 147 and subtilisin 168 described in WO 89/06279 and protease PD138 described in (WO 93/18140). Other useful proteases may be those described in WO 92/175177, WO 01/016285, WO 02/026024 and WO 02/016547. Examples of trypsin-like proteases are trypsin (e.g. of porcine or bovine origin) and the Fusarium protease described in WO 89/06270, WO 94/25583 and WO 05/040372, and the chymotrypsin proteases derived from Cellumonas described in WO 05/052161 and WO 05/052146.

Most preferably the protease is a subtilisins (EC 3.4.21.62).

Examples of subtilases are those derived from Bacillus such as Bacillus lentus, B. alkalophilus, B. subtilis, B. amyloliquefaciens, Bacillus pumilus and Bacillus gibsonii described in; US7262042 and WO09/021867, and subtilisin lentus, subtilisin Novo, subtilisin Carlsberg, Bacillus licheniformis, subtilisin BPN', subtilisin 309, subtilisin 147 and subtilisin 168 described in WO89/06279 and protease PD138 described in (WO93/18140). Preferably the subsilisin is derived from Bacillus, preferably Bacillus lentus, B. alkalophilus, B. subtilis, B. amyloliquefaciens, Bacillus pumilus and Bacillus gibsonii as described in US 6,312,936 B1, US 5,679,630, US 4,760,025, US7,262,042 and WO 09/021867. Most preferably the subtilisin is derived from Bacillus gibsonii or Bacillus Lentus.

Suitable commercially available protease enzymes include those sold under the trade names Alcalase^{®}, Blaze^{®}; DuralaseTm, DurazymTm, Relase^{®}, Relase^{®} Ultra, Savinase^{®}, Savinase^{®} Ultra, Primase^{®}, Polarzyme^{®}, Kannase^{®}, Liquanase^{®}, Liquanase^{®} Ultra, Ovozyme^{®}, Coronase^{®}, Coronase^{®} Ultra, Neutrase^{®}, Everlase^{®} and Esperase^{®} all could be sold as Ultra^{®} or Evity^{®} (Novozymes A/S).

The composition may use cutinase, classified in EC 3.1.1.74. The cutinase used according to the invention may be of any origin. Preferably cutinases are of microbial origin, in particular of bacterial, of fungal or of yeast origin.

Suitable amylases (alpha and/or beta) include those of bacterial or fungal origin. Chemically modified or protein engineered mutants are included. Amylases include, for example, alpha-amylases obtained from *Bacillus*, e.g. a special strain of *B*. *licheniformis*, described in more detail in GB 1,296,839, or the *Bacillus* sp. strains disclosed in WO 95/026397 or WO 00/060060. Commercially available amylases are Duramyl^{™}, Termamyl^{™}, Termamyl Ultra^{™}, Natalase^{™}, Stainzyme^{™}, Amplify^{™}, Fungamyl^{™} and BAN^{™} (Novozymes A/S), Rapidase^{™} and Purastar^{™} (from Genencor International Inc.).

Suitable cellulases include those of bacterial or fungal origin. Chemically modified or protein engineered mutants are included. Suitable cellulases include cellulases from the genera *Bacillus*, *Pseudomonas*, *Humicola*, *Fusarium*, *Thielavia*, *Acremonium*, e.g. the fungal cellulases produced from *Humicola insolens*, *Thielavia terrestris*, *Myceliophthora thermophila*, and *Fusarium oxysporum* disclosed in US 4,435,307, US 5,648,263, US 5,691,178, US 5,776,757, WO 89/09259, WO 96/029397, and WO 98/012307. Commercially available cellulases include Celluzyme^{™}, Carezyme^{™}, Celluclean ^{™}, Endolase^{™}, Renozyme^{™} (Novozymes A/S), Clazinase^{™} and Puradax HA^{™} (Genencor International Inc.), and KAC-500(B)^{™} (Kao Corporation). Celluclean^{™} is preferred.

Suitable peroxidases/oxidases include those of plant, bacterial or fungal origin. Chemically modified or protein engineered mutants are included. Examples of useful peroxidases include peroxidases from *Coprinus*, e.g. from *C*. *cinereus*, and variants thereof as those described in WO 93/24618, WO 95/10602, and WO 98/15257. Commercially available peroxidases include Guardzyme^{™} and Novozym^{™} 51004 (Novozymes A/S).

Further enzymes suitable for use are discussed in WO 2009/087524, WO 2009/090576, WO 2009/107091, WO 2009/111258 and WO 2009/148983.

### Enzyme Stabilizers

Any enzyme present in the composition may be stabilized using conventional stabilizing agents, e.g., a polyol such as propylene glycol or glycerol, a sugar or sugar alcohol, lactic acid, boric acid, or a boric acid derivative, e.g., an aromatic borate ester, or a phenyl boronic acid derivative such as 4-formylphenyl boronic acid, and the composition may be formulated as described in e.g. WO 92/19709 and WO 92/19708.

### Chelating Agent

Chelating agents may be present or absent from the detergent compositions.

If present, then the chelating agent is present at a level of from 0.01 to 5 wt.%.

Preferred chelating agents are phosphonic acid (or salt thereof) chelating agents, preferably selected from: 1-Hydroxyethylidene-1,1-diphosphonic acid (HEDP); Diethylenetriaminepenta(methylenephosphonic acid) (DTPMP); Hexamethylenediaminetetra(methylenephosphonic acid) (HDTMP); Aminotris(methylenephosphonic acid) (ATMP); Ethylenediaminetetra(methylenephosphonic acid) (EDTMP); Tetramethylenediaminetetra(methylenephosphonic acid) (TDTMP); and, Phosphonobutanetricarboxylic acid (PBTC).

### Perfume/Fragrances

As used herein the terms fragrance and perfume are used interchangeably.

The composition preferably comprises a fragrance. Many suitable examples of fragrances are provided in the CTFA (Cosmetic, Toiletry and Fragrance Association) 1992 International Buyers Guide, published by CFTA Publications and OPD 1993 Chemicals Buyers Directory 80th Annual Edition, published by Schnell Publishing Co.

Preferably the laundry composition comprises a fragrance, preferably wherein the fragrance is included between 0.001 and 2.0wt%, more preferably 0.01 and 1.5wt% and most preferably 0.1 and 1.0wt%. Preferably the fragrance comprises greater than 50wt% biodegradable materials, more preferably greater than 60wt% biodegradable materials, more preferably greater than 70wt% biodegradable materials, more preferably greater than 80wt% biodegradable materials, more preferably greater than 90% biodegradable materials and most preferably the fragrance consists of 100wt% biodegradable materials.

Preferably the fragrance comprises at least one note (compound) from: alpha-isomethyl ionone, benzyl salicylate; citronellol; coumarin; hexyl cinnamal; linalool; pentanoic acid, 2-methyl-, ethyl ester; octanal; benzyl acetate; 1,6-octadien-3-ol, 3,7-dimethyl-, 3-acetate; cyclohexanol, 2-(1,1-dimethylethyl)-, 1-acetate; delta-damascone; beta-ionone; verdyl acetate; dodecanal; hexyl cinnamic aldehyde; cyclopentadecanolide; benzeneacetic acid, 2-phenylethyl ester; amyl salicylate; beta-caryophyllene; ethyl undecylenate; geranyl anthranilate; alpha-irone; beta-phenyl ethyl benzoate; alpa-santalol; cedrol; cedryl acetate; cedry formate; cyclohexyl salicyate; gamma-dodecalactone; and, beta phenylethyl phenyl acetate.

### Polymers

The composition may comprise one or more further polymers. Examples are carboxymethylcellulose, poly (ethylene glycol), poly(vinyl alcohol), polycarboxylates such as polyacrylates, maleic/acrylic acid copolymers and lauryl methacrylate/acrylic acid copolymers.

### Optional Ingredients

A composition of the invention may contain optional ingredients to enhance performance and / or consumer acceptability. Examples of such ingredients include anti-foams, fluorescers, shading dyes, preservatives, anti-microbials (e.g. bactericides), foam boosting agents, polyelectrolytes, anti-shrinking agents, anti-wrinkle agents, anti-oxidants, sunscreens, anticorrosion agents, drape imparting agents, anti-static agents, ironing aids, dyes / colorants, shading dyes, pearlisers and/or opacifiers and microcapsules. Each of these ingredients will be present in an amount effective to accomplish its purpose. Generally, these optional ingredients are included individually at an amount of up to 5% (by weight based on the total weight of the composition).

If an anti-foam is included it is preferably a fatty acid soap. Suitable fatty acids in the context of this invention include aliphatic carboxylic acids of formula RCOOH, where R is a linear or branched alkyl or alkenyl chain containing from 6 to 24, more preferably 10 to 22, most preferably from 12 to 18 carbon atoms and 0 or 1 double bond. Preferred examples of such materials include saturated C12-18 fatty acids such as lauric acid, myristic acid, palmitic acid or stearic acid; and fatty acid mixtures in which 50 to 100% (by weight based on the total weight of the mixture) consists of saturated C12-18 fatty acids. Such mixtures may typically be derived from natural fats and/or optionally hydrogenated natural oils (such as coconut oil, palm kernel oil or tallow).

### Examples

The invention will be demonstrated by the following non-limiting examples.

The reference to MorvLip is an enzyme from *Moritella viscosa* having the sequence of SEQ. ID. 1.

The reference to PinLip is an enzyme from *Psychromonas ingrahamii* as described in WO 2017/036901.

### Production of MorvLip

### Materials and methodology

The gene encoding the protein of interest, MorvLip, with a C-terminal poly-histidine tag (His₆ tag), was obtained from TWIST Biosciences, San Francisco, in a pET28a vector (with kanamycin resistance). MorvLip-pET28a was transformed into *E. coli* BL21 (DE3) cells. Positive colonies from the transformation were used to over-express MorvLip in 1 L LB media cultures in 2.5 L Tunair^{®} flasks for 39 hours at 12 °C after IPTG (1 mM). Cell paste was collected and stored at -20 °C before use. Cells were lysed and purified using Ni-IMAC chromatography, then dialysed into storage buffer. Protein concentration was determined with a Qubit^{™} 4 Fluorometer (Thermo Fisher Scientific) and Qubit^{™} Protein Assay Kit. Protein samples were then stored at 4 °C for up to 3 months prior to use in wash studies.

### Buffers

Purification buffers (shown in Table 1) were filtered through a 0.2 µM nylon membrane filter (Whatman, Maidstone, UK) by vacuum. Buffers were cooled to 4 °C prior to pH adjustment.

HEPPS is 4-(2-Hydroxyethyl)-1-piperazinepropanesulfonic acid, 4-(2-Hydroxyethyl)piperazine-1-propanesulfonic acid, *N*-(2-Hydroxyethyl)piperazine-*N*'-(3-propanesulfonic acid)
SLES is sodium lauryl ether sulfate
TCEP is tris(2-carboxyethyl)phosphine
EDTA is ethylenediaminetetraacetic acid

**Table 1. Buffers used for purification of MorvLip.**

| | |
|---|---|
| Immobilised Metal Affinity | 50 mM HEPPS pH 8.0 |
| Chromatography (IMAC) Buffer A | 1 M Sodium Chloride |
| | 50 mM Maltose |
| | 0.005 % (w/v) SLES |
| | 0.3 mM TCEP |
| | 20 mM Imidazole |
| Immobilised Metal Affinity | 50 mM HEPPS pH 8.0 |
| Chromatography (IMAC) Buffer B | 1 M Sodium Chloride |
| | 50 mM Maltose |
| | 0.005 % (w/v) SLES |
| | 0.3 mM TCEP |
| | 250 mM Imidazole |
| Storage Buffer | 50 mM HEPPS pH 8.0 |
| | 1 M Sodium Chloride |
| | 50 mM Maltose |
| | 5 mM EDTA |
| | 0.005 % (w/v) SLES |
| | 0.3 mM TCEP |

### Transformation

An aliquot of *E. coli* BL21 (DE3) (50 µL) was transferred from the -80 °C freezer onto ice and allowed to thaw for 5 minutes. Plasmids encoding the gene of interest were mixed with cells (1 µL for plasmids with 100 ng/µL concentration). One or two plasmids were added for single or double transformations, respectively, were then added to the cells with gentle mixing and left on ice for 30 minutes. Cells were heat-shocked at 42 °C in a water bath for 45 seconds before returning to ice for a further 5 minutes. Pre-warmed (37 °C) SOC media (200 µL) was added to the cells before incubating at 37 °C and 220 rpm for one hour. The transformed cells were then plated onto LB agar plates which had been supplemented with kanamycin (50 mg/L). Plates were incubated at 37 °C overnight, or at 20°C over 3 nights. Non-transformed cells were used as a negative control, whilst control plasmids pUC18 or pUC19 were used as a positive control.

### Over-expression

Pre-warmed (37 °C) LB media (1 L), starter culture (10 mL), and kanamycin (50 mg/L) were added to a sterile fully baffled 2.5 L Tunair^{™} flask and incubated at 37 °C with 250 rpm until OD₆₀₀ reached: 0.6-0.8. Flasks were then placed on ice for 20-30 minutes, after which β-D-1-thiogalactopyranoside (IPTG) was added to 1 mM final concentration. The flasks were then incubated at 12 °C for 39 h. Cells were harvested by centrifugation at 4500 rpm at 4 °C for 20 minutes. Cell pellets were washed by resuspension in phosphate buffered saline. The resuspension was decanted into 50 mL sterile Falcon tubes and the cells we pelleted by centrifugation at 4500 rpm at 4 °C for 20 minutes. Supernatant liquid was discarded, and cell pellets were stored at -20 °C.

### Sonication

Lysis buffer was prepared by adding Benzonaze^{®} (1U/50 mL) and lysozyme (0.1 mg/mL) to IMAC buffer A. Cell paste (2-15 g) was transferred from -20 °C storage onto ice. Appropriate ice-cold lysis buffer was added (20-50 mL), and the cells were thawed with occasional inversion of the Falcon tubes to start resuspension of the cells. After the cells were thawed, a metal spatula was used to vigorously mix the cells until a homogeneous suspension was attained. The cell suspension was decanted into a narrow 75 mL glass beaker and placed securely in a bed of wet ice. The cell suspension was then sonicated with a Fisherbrand^{™} 120 dismembrator (Fisher Scientific, Loughborough) with a 6.3 mm probe. The programme was set at 80 % intensity with a pulse of 5 seconds ON, 10 seconds **OFF** for a total sonication time of 10 minutes to prevent heating of the sample. The sonicated cells were centrifuged with 14500 rpm at 4 °C for 30 minutes, after which the supernatant containing soluble protein was separated from the pelleted insoluble fractions for downstream purification. The supernatant fraction was then clarified using a 28 mm membrane syringe filter with Sartorius Stedim, minisart 0.2 µm single-use filtration unit (Stonehouse, UK).

### Purification

Protein samples were purified using an ÄKTA purifier (GE Healthcare, Cincinnati, OH, USA) kept in a cold room at 7 °C.

### Nickel-NTA affinity chromatography

A 5 mL nickel-NTA (Ni-NTA) column (Cytiva) which had been stored in 20 % (v/v) ethanol was equilibrated with 10 column volumes (CV) of ddH₂O before equilibration with 10 CV of IMAC buffer A. Clarified supernatant was then loaded and the flow through was collected, where proteins of interest were expected to bind to the Ni-NTA resin until elution buffer was applied. The column was then washed with 20 CV of IMAC buffer A, or until the absorbance at 280 nm (A₂₈₀) returned near to the baseline of the lysis buffer. The protein of interest was then eluted with 5 CV of IMAC buffer B. Fractions were analysed by SDS-PAGE and Western blot. Fractions containing the gene of interest were combined for downstream processing and experiments.

### Dialysis

Dialysis tubing membrane (Thermo Fisher Scientific) with a molecular weight cut-off (MWCO) of 8 kDa was used to dialyse soluble protein samples. Membranes were rinsed with ddH₂O, a knot was tied to seal the tubing at one end and protein sample was added. Another knot was tied to fully seal the tubing, and plastic clips were attached to both ends. The tubing was carefully placed into 4 L of Storage buffer at 4 °C and left with gentle stirring for 18 hours.

### Protein concentration determination

Protein concentration was determined with a Qubit^{™} 4 Fluorometer (Thermo Fisher Scientific) and Qubit^{™} Protein Assay Kit using the manufacturers recommended conditions. The Qubit^{™} protein assay works by reading fluorescence of a detergent-based dye which responds quantitively to the presence of protein in solution.

Before performing the assay, all reagents were equilibrated to room temperature. Each time an assay was performed, a calibration was performed using three protein standards provided in the kit. Qubit^{™} working solution was prepared by diluting Qubit^{™} protein reagent 1:200 in Qubit^{™} protein buffer in a clean plastic tube. Calibration standards were prepared by addition of 190 µL working solution to 10 µL Protein Standards 1, 2, and 3. Protein samples were prepared by addition of 190 µL working solution to 10 µL protein sample. All tubes were vortexed for 2-3 seconds before leaving to incubate for 15 minutes. The Qubit^{™} 4 Fluorometer was then calibrated with the three protein standards before reading each protein sample.

### Protein purity determination by SDS-PAGE analysis

A Bio-Rad mini-Protean cell was employed for SDS-PAGE. Protein samples were boiled for 10 minutes at 100 °C after dilution in Laemmli loading buffer²⁵⁷ to yield denatured samples which were ready to load after centrifugation at 12000 RPM for 5 minutes. After cooling to RT, samples (2-20 µL) were loaded onto a 4-12% ExpressPlus^{™} PAGE Gels (GenScript, Piscatawat, NJ, USA). 2 µL of Spectra Broad range multicolour protein ladder (Bio-Rad,) was loaded to the gel to enable determination of protein molecular weights.

MOPS running buffer (50 mM Tris-Base, 50 mM MOPS, 1 mM EDTA, and 0.1 % SDS (w/v), pH 7.7) and electrode conditions of 140 V, 400 mA, 60 minutes at RT, were employed to achieve protein separation. Quick Coomassie Stain (Generon, Slough, UK) was used to stain protein bands on SDS-PAGE gels following the manufacturer's protocol. SDS-PAGE gels were transferred into ddH₂O at RT for 1 hour, the ddH₂O was replenished and the gel was left for a further 1 hour to remove all unbound dye.

### Protein characterisation by Western-blot analysis

SDS-PAGE gels were prepared with the samples of interest without staining the gel following protein separation.

Protein bands on the SDS-PAGE gel were transferred to a nitrocellulose membrane (Sartorius Stedim) in a Pierce G2 Fast Blotter (Thermo Fisher Scientific) (25 V, 1.3 A, 7 minutes) following manufacturer's instructions. Antibody applications and washes were conducted using the iBind Western system (Thermo Fisher Scientific). The iBind Western system takes advantage of lateral flow capillary diffusion to perform all Western Blot steps in a single procedure.

1X iBind master mix was prepared by the addition of 5X iBind Buffer (6 mL), additive (300 µL), and ddH₂O (23.7 mL) into a sterile 50 mL Falcon tube. Nitrocellulose membranes were soaked in iBind master mix (6 mL) for 5 minutes. Meanwhile an iBind card was pre-equilibrated with 6 mL iBind master mix, without liquid touching the cartridge. Antibody solutions were prepared following the manufacturer's advised dilution in the iBind master mix to a final volume of 2 mL. After the addition additional iBind master mix (1 mL) to the centre of the card, the soaked nitrocellulose membrane was placed so that protein bands were in contact with the card, and the lowest molecular weight bands were at the bottom. The iBind system was then closed and the following solutions were added to slots:
1. 2 mL 1X primary antibody solution
2. 2 mL iBind master mix
3. 2 mL 1X secondary antibody solution
4. 6 mL iBind master mix

Monoclonal Anti-poly-histidine Mouse (Sigma) and IRDye^{®} 800CW Goat anti-Mouse IgG (LI-COR) were used as the primary and secondary antibodies, respectively.

The iBind system was left to incubate at RT between 4 and 18 hours. The nitrocellulose membrane was then rinsed in ddH₂O to remove any residual unbound antibodies. The membrane was then imaged using an Odyssey CLx Imaging System (LI-COR, Lincoln, NE, USA), and analysed using Image Studio^{™} Lite Light (LI-COR).

Identity of the full length sequence of MorvLip was confirmed by SDS-PAGE iBind Western-blot analysis and purity of the MorvLip was established by SDS-PAGE analysis of the MorvLip Ni-IMAC purification fractions. This analysis indicated the satisfactory production and purification of the MorvLip enzyme.

### Example 1 - Wash application testing: comparison of MorvLip and Lipex^{®} Evity^{®} 200L

### Materials and methodology

MorvLip was compared to the commercial enzyme Lipex^{®} Evity^{®} 200L (Novozymes) for cleaning efficacy. Due to the large quantity of protein needed in this study (~100 mg) the comparison with PinLip was unviable due to its low overexpression and purification yields. The stained fabric set "Lipase set 2" was employed to determine stain removal for a wide range of stains. Pre-soaking fabrics with particularly tough stains can increase the cleaning efficacy of laundry formulations and is a consumer habit. Therefore, the efficacy of MorvLip was explored using a pre-soak method. All stained fabrics were pre-soaked overnight in the bespoke formulation F4 at 2.5 g/L with either no enzyme or 125 mg/L of MorvLip, or Lipex^{®} Evity^{®} 200L, and 50 mM Tris-HCI (pH 8.5). The pre-soaked stained fabrics were then washed in a Tergo-O-Tometer with 0.1 g/L Persil UK Non-Bio at 30 °C for 45 minutes, followed by a rinse in water. 0.1 g/L of detergent is at the lower threshold for a laundry cycle and was chosen to highlight the positive effect of using lipases in formulation to reduce the amount of detergent required for effective stain removal. Washed fabric stains were then measured to determine the stain removal index (SRI) for each pre-soak treatment method.

### Pre-soak method

Before performing the Tergo-O-Tometer wash on stained fabrics, a pre-soak was performed using bespoke formulation alone, with MorvLip, or Lipex^{®} Evity^{®} 200L. Working pre-soak solutions were prepared by adding bespoke formulation 2X (200 mL), water (150 mL), and 50 mL MorvLip 8X, Lipex^{®} Evity^{®} 200L 8X, or water, in that order. Standardised swatches of knitted polyester with oil-based stains (Lipase set 2, Warwick, Equest) were fully submerged in 100 mL of each of the working pre-soak solutions in quadruplicate and left overnight.

**Table 2. Solutions required for pre-soaking wash method.**

| **Solution** | **Components** |
|---|---|
| Bespoke formulation 2X | 50 mM Tris-HCI pH 8.5 |
| | 5 g/L RL2:CAPB 2:1 |
| MorvLip 8X | 1 mg/mL MorvLip |
| | 50 mM HEPPS pH 8.0 |
| | 1 M NaCl |
| | 5 mM EDTA |
| | 50 mM maltose |
| Lipex^{®} Evity 200L 8X | 1 mg/mL Lipex^{®} Evity 200L |
| | 50 mM HEPPS pH 8.0 |
| | 1 M NaCl |
| | 5 mM EDTA |
| | 50 mM maltose |

Bespoke formulation 2X is a pH 8.5 buffered di-rhamnolipid (sourced from Evonik) cocamidopropyl betaine surfactant composition (2:1 weight ratio) dosed at 5g/L.

### Terg-O-Tometer wash performance

A Terg-O-Tometer (Testfabric Inc., West Pittson, PA, US) was then employed to simulate real-life washing conditions found in washing machines. The Terg-O-Tometer is a lab-scale washing device used to evaluate the cleaning efficacy of laundry washing liquids and powders. The instrument is an agitator-type washer with 6 beakers under temperature control so that experiments can be conducted with replicates easily. Each wash was performed using a combination of stained fabric and a mixture of clean woven cotton squares and knitted polyester squares (10x10 cm) called ballast. 1 L wash liquor was used to wash each sheet of fabric. After washing, the stained fabrics were removed from the wash liquor, and the ballast fabric was discarded. The remaining stained fabrics were then rinsed in 1L of water and allowed to dry on a horizontal rack at room temperature overnight in darkness. Dried stain fabrics were then measured in an X-Rite instrument (Pantone, Carlstadt, NJ, US) with a UV excited light source to determine ΔE and SRI relative to unwashed white fabric.

### SRI calculation

All fabric stain removals were assessed using the Stain Removal Index (SRI) method. Stained fabrics were digitally scanned to measure their colour difference to a control white fabric using the equation: $ΔE = {\left[{\left(ΔL\right)}^{2}+{\left(Δa\right)}^{2}+{\left(Δb\right)}^{2}\right]}^{\frac{1}{2}}$

Where ΔL, Δa, and Δb are the difference in darkness, redness, and yellowness, respectively, between a white fabric and the stained fabric.

Eq. 1 tells us that lower values of ΔL, Δa, and Δb will result in lower values of ΔE, wherein a value of 0 would correspond to a completely white fabric.

The efficacy of cleaning is then expressed as: $SRI = 100 - ΔE$

Wherein higher SRI values correspond to cleaner fabric, and 100 corresponds to completely white fabric.

The raw values from the results of equations 1 & 2 were normalised to give the unstained fabric being 100. The results for the margarine fat-stained fabric are given below in table 3. There were 4 replicates.

**Table 3**

| | **Water** | **Lipex^{®} Evity 200L** | **MorvLip** |
|---|---|---|---|
| **Unstained Fabric** | 100 | 100 | 100 |
| **Margarine** | 62.46 ± 2.36 | 68.52 ± 1.76 | 78.27 ± 6.69 |

As can be seen the MorvLip performs much better than the commercial lipase (Lipex^{®} Evity) on the fatty margarine stain in terms of stain removal.

### Example 2 - Stability in commercial additives for the cold adapted lipases

### Materials and methodology

This experiment shows that the stability of MorvLip versus PinLip as measured by residual efficacy is greater when the lipase is present in a formulation containing certain useful general laundry composition additives.

MorvLip is a quite different enzyme sequence that to PinLip. The MorvLip of SEQ. ID. 1 of this specification shares only 35% sequence identity with the PinLip enzyme of WO 2017/036901.

MorvLip, and PinLip were tested for long-term stability in the presence of a soil-releasing polymer (SRP), and a protease, which are commonly used in commercial laundry formulations. The enzymes were incubated at 1 mg/mL in the presence of 2% SRP, or 0.95 mg/mL protease, in SEC buffer (50 mM HEPPS pH 8.0, 1 M Sodium Chloride, 50 mM Maltose, 5 mM EDTA, 0.005 % (w/v) SLES, 0.3 mM TCEP) for 4 weeks. *P*Np-laurate assays were performed to determine the hydrolytic activity. The initial activity was recorded in the absence of any additive, and then after 4 weeks the activity was measured again. The residual activity after 4 weeks was calculated as a percentage of the initial activity. All activity assays were performed in 50 mM Tris-HCI pH 8.6 at 25 °C.

### PNp-laurate hydrolysis assay

pNp laurate was prepared as 8 mM stock solutions in methanol (51.4 mg/20 mL) and stored at -20 °C for future use. Stock solutions were diluted to 0.25 mM in ddH₂O, protected from light exposure by aluminium foil, and kept on ice. All solutions were used within 24 hours of dilution. Diluted *p*Np ester was added to a reservoir and allowed to equilibrate to the desired temperature. The TECAN Infinite M200 PRO plate reader was switched on at least 15 minutes prior to the experiment. Diluted proteins samples were prepared by dilution of protein samples (1 mg/mL) 10-fold in Tris-HCI buffer (100 mM, pH 8.6). Samples were prepared by the addition of 100 mM Tris-HCI pH 8.0 (100 µL), and diluted protein solution (20 µL) to a 96-well microtiter plate in triplicate. ddH₂O (20 µL) was added in replacement of protein solution in triplicate as a control, this serves to measure background *p*Np ester hydrolysis. In the case where different additives are employed, each additive is added in the absence of protein to measure each individual background activity. The plate was left to equilibrate to the temperature of the plate reader for 5 minutes. Using a 12 channel P100 pipette, 0.25 mM *p*Np laurate (80 µL) was added to all wells, and the plate was transferred into the plate reader. Activity was tracked by detection of absorption at 347 nm (A₃₄₇) every 60 seconds over 10 minutes. Enzymatic steady-state hydrolysis rate of *p*Np esters were calculated in PRISM 8.0.

For kinetic measurements in a wide range of pH conditions, the method of pH-independent described by Lucia *et. al* was adapted. For the set-up of samples, the same methodology was followed as described in the previous section, except the buffer was interchangeable with any buffer system. Activity was measured by detection of absorption at 347 nm (A₃₄₇) every 60 seconds over 10 minutes. Enzymatic hydrolysis rate of *p*Np-laurate were calculated in excel and PRISM 8.0.

The results of the stability experiment are given in table 4. There were 3 replicates.

**Table 4 - Relative activity (%) after 4 weeks incubation as a percentage of the 'No additive' condition**

| | **MorvLip** | **PinLip** |
|---|---|---|
| **No Additive** | 100 | 100 |
| (SRP) TexCare^{®} UL 50 | 40.23 ± 4.91 | 22.67 ± 1.59 |
| (P) CE16L | 34.92 ± 4.47 | 6.91 ± 2.17 |

TexCare^{®} UL 50 corresponds to the soil release polymer. TexCare^{®} soil release polymers are commercially available from Clariant. CE16L corresponds to the protease and relates to Carnival Evity 16L, commercially available from Novozymes).

The MorvLip lipase retained much higher activity compared to PinLip when incorporated in compositions containing usual laundry detergent additives such as protease and soil release polymer.

### Example 3 - Residual stability after 4 weeks as a percentage of initial activity

The stability of MorvLip, PinLip, Lipex^{®} 100L, and Lipex^{®} Evity^{®} 200L in buffer over 4 weeks was tested. Residual activity given as a percentage of the initial activity after 4 weeks is shown in table 5.

**Table 5 - Residual activity (%) after 4 weeks incubation as a percentage of the activity on day 0**

| **MorvLip** | **PinLip** | **Lipex^{®} 100** | **Lipex^{®} Evity 200L** |
|---|---|---|---|
| 101.80 ± 6.56 | 36.00 ± 0.88 | 103.32 ± 4.45 | 105.71 ± 5.69 |

The MorvLip, and the two Lipex^{®} enzymes broadly retained their initial activity levels, however the MorvLip enzyme retained much higher activity levels versus the PinLip lipase enzyme.

## Claims

1. A detergent composition comprising:
(a) from 0.0005 to 6 wt.%, preferably from 0.005 to 4 wt.%, more preferably from 0.001 to 2 wt.% of a lipase from *Moritella viscosa*; and,
(b) from 1 to 60 wt.% preferably from 1 to 50 wt.%, more preferably from 1 to 35 wt.% of a detersive surfactant, wherein the lipase from *Moritella viscosa* has a sequence identity of at least 70% with SEQ. ID. 1.

2. A detergent composition according to claim 1, wherein the lipase from *Moritella viscosa* has a sequence identity of at least 75%, more preferably 80%, more preferably 85%, even more preferably 90%, even more preferably 95%, even more preferably 98%, even more preferably 99%, most preferably 100% with SEQ. ID. 1.

3. A detergent composition according to claim 1 or claim 2, wherein the detergent composition comprises anionic and/or nonionic surfactant, preferably comprising both anionic and nonionic surfactant.

4. A detergent composition according to claim 3, wherein the anionic surfactant is present at a level of from 1 to 50 wt.%, preferably from 2 to 40 wt.%, more preferably from 3 to 30 wt.% and is preferably selected from linear alkyl benzenesulphonate, secondary alkane sulphonate, sodium laureth ether sulphate, sodium lauryl sulphate, sodium oleyl sulphate and sodium oleyl ether sulphate, methyl ester sulphonate, secondary alkyl sulphate (SALS), cardanol ether sulphate and a rhamnolipid.

5. A detergent composition according to claim 3 or claim 4, wherein the nonionic surfactant is present at a level of from 1 to 30 wt.%, preferably from 2 to 20 wt.%, more preferably from 3 to 15 wt.% and is preferably selected from an alcohol ethoxylate, an alcohol propoxylate, a methyl ester ethoxylate and an alkyl poly glycoside.

6. A detergent composition according to any preceding claim, wherein the detergent composition is a laundry detergent composition.

7. A laundry detergent composition according to claim 6, wherein the laundry detergent composition is in the form of a liquid, solid, powder, pastille, bead or paste, preferably the composition is a liquid or a powder, more preferably a liquid detergent.

8. A laundry detergent composition according to claim 6 or claim 7, wherein the laundry detergent composition comprises an alkoxylated polyamine, preferably at a level of from 0.1 to 8 wt.%, more preferably from 0.2 to 6 wt.%, most preferably from 0.5 to 5 wt.%.

9. A laundry detergent composition according to any one of claims 6 to 8, wherein the laundry detergent composition comprises soil release polymer, the soil release polymer preferably selected from copolyesters of dicarboxylic acids and polydiols, more preferably a copolyester formed by condensation of terephthalic acid ester and 1,2-propanediol, the soil release polymer preferably present at a level of from 0.1 to 8 wt.%, more preferably from 0.2 to 6 wt.%, most preferably from 0.5 to 5 wt.%.

10. A detergent composition according to any preceding claim, additionally comprising one or more further enzymes selected from the group consisting of: proteases, cellulases, alpha-amylases, peroxidases/oxidases, pectate lyases, and/or mannanases, preferably the one or more enzymes comprises protease.

11. A method of treatment of a textile having a fatty stain, wherein said textile is treated with a composition according to any one of claims 1 to 10, to provide enhanced lipolytic cleaning against fatty stains, preferably margarine stains, said textile preferably subsequently rinsed and dried.

12. A method of treatment of a textile having a fatty stain, wherein said textile is pre-treated with a composition according to claim 1 or claim 2, to provide enhanced lipolytic cleaning against fatty stains, preferably margarine stains, said textile then subsequently washed with a laundry main wash composition, and then preferably subsequently rinsed and dried.

13. Use of a lipase from *Moritella viscosa* having a sequence identity of at least 70% with SEQ. ID. 1, to improve cleaning of fatty stains, preferably margarine stains on textiles.

14. Use of a detergent composition comprising a lipase from *Moritella viscosa* having a sequence identity of at least 70% with SEQ. ID. 1, to improve cleaning of fatty stains, preferably margarine stains on textiles.

15. Use according to either claim 13 or claim 14, wherein the lipase from *Moritella viscosa* has a sequence identity of at least 75%, more preferably 80%, more preferably 85%, even more preferably 90%, even more preferably 95%, even more preferably 98%, even more preferably 99%, most preferably 100% with SEQ. ID. 1.

## Patentansprüche

1. Reinigungsmittelzusammensetzung, umfassend:
(a) 0,0005 bis 6 Gew.-%, vorzugsweise 0,005 bis 4 Gew.-%, bevorzugter 0,001 bis 2 Gew.-% einer Lipase aus Moritella viscosa; und
(b) 1 bis 60 Gew.-%, vorzugsweise 1 bis 50 Gew.-%, bevorzugter 1 bis 35 Gew.-% eines Reinigungsmitteltensids, wobei die Lipase aus Moritella viscosa eine Sequenzidentität von mindestens 70% mit der SEQ. ID.1 aufweist.

2. Reinigungsmittelzusammensetzung nach Anspruch 1, wobei die Lipase aus Moritella viscosa eine Sequenzidentität von mindestens 75%, bevorzugter 80%, bevorzugter 85%, noch bevorzugter 90%, noch bevorzugter 95%, noch bevorzugter 98%, noch bevorzugter 99% und höchst bevorzugt 100% mit der SEQ.ID.1 aufweist.

3. Reinigungsmittelzusammensetzung nach Anspruch 1 oder Anspruch 2, wobei die Reinigungsmittelzusammensetzung anionisches und/oder nichtionisches Tensid, vorzugsweise sowohl anionisches als auch nichtionisches Tensid umfasst.

4. Reinigungsmittelzusammensetzung nach Anspruch 3, wobei das anionische Tensid in einer Menge von 1 bis 50 Gew.-%, vorzugsweise von 2 bis 40 Gew.-%, bevorzugter von 3 bis 30 Gew.-% vorliegt und vorzugsweise unter linearem Alkylbenzolsulfonat, sekundärem Alkansulfonat, Natriumlaurethethersulfat, Natriumlaurylsulfat, Natriumoleylsulfat und Natriumoleylethersulfat, Methylestersulfonat, sekundärem Alkylsulfat (SALS), Cardanol-Ethersulfat und einem Rhamnolipid ausgewählt ist.

5. Reinigungsmittelzusammensetzung nach Anspruch 3 oder Anspruch 4, wobei das nichtionische Tensid in einer Menge von 1 bis 30 Gew.-%, vorzugsweise von 2 bis 20 Gew.-%, bevorzugter von 3 bis 15 Gew.-% vorliegt und vorzugsweise unter einem Alkoholethoxylat, einem Alkoholpropoxylat, einem Methylesterethoxylat und einem Alkylpolyglykosid ausgewählt ist.

6. Reinigungsmittelzusammensetzung nach einem vorhergehenden Anspruch, wobei die Reinigungsmittelzusammensetzung eine Waschmittelzusammensetzung ist.

7. Waschmittelzusammensetzung nach Anspruch 6, wobei die Waschmittelzusammensetzung in Form einer Flüssigkeit, eines Feststoffs, eines Pulvers, einer Pastille, von Kügelchen oder einer Paste vorliegt, wobei die Zusammensetzung vorzugsweise eine Flüssigkeit oder ein Pulver ist, bevorzugter ein flüssiges Reinigungsmittel.

8. Waschmittelzusammensetzung nach Anspruch 6 oder Anspruch 7, wobei die Waschmittelzusammensetzung ein alkoxyliertes Polyamin umfasst, vorzugsweise in einer Menge von 0,1 bis 8 Gew.-%, bevorzugter von 0,2 bis 6 Gew.-%, höchst bevorzugt von 0,5 bis 5 Gew.-%.

9. Waschmittelzusammensetzung nach irgendeinem der Ansprüche 6 bis 8, wobei die Waschmittelzusammensetzung ein Soil-Release-Polymer umfasst, wobei das Soil-Release-Polymer vorzugsweise unter Copolyestern von Dicarbonsäuren und Polydiolen ausgewählt ist, bevorzugter unter einem durch Kondensation von Terephthalsäureester und 1,2-Propandiol gebildeten Copolyester, wobei das Soil-Release-Polymer vorzugsweise in einer Menge von 0,1 bis 8 Gew.-%, bevorzugter von 0,2 bis 6 Gew.-% und höchst bevorzugt von 0,5 bis 5 Gew.-% vorliegt.

10. Reinigungsmittelzusammensetzung nach einem vorhergehenden Anspruch, die zusätzlich ein oder mehrere weitere Enzyme umfasst, ausgewählt aus der Gruppe, bestehend aus: Proteasen, Cellulasen, Alpha-Amylasen, Peroxidasen/ Oxidasen, Pektat-Lyasen und/oder Mannanasen, wobei das eine oder die mehreren Enzyme vorzugsweise Protease umfassen.

11. Verfahren zur Behandlung eines Textils mit Fettflecken, wobei das Textil mit einer Zusammensetzung nach irgendeinem der Ansprüche 1 bis 10 behandelt wird, um eine verbesserte lipolytische Reinigungswirkung bei Fettflecken, vorzugsweise Margarineflecken, zu erzielen, wobei das Textil vorzugsweise anschließend gespült und getrocknet wird.

12. Verfahren zur Behandlung eines Textils mit Fettflecken, wobei das Textil mit einer Zusammensetzung nach Anspruch 1 oder Anspruch 2 vorbehandelt wird, um eine verbesserte lipolytische Reinigungswirkung bei Fettflecken, vorzugsweise Margarineflecken, zu erzielen, wobei das Textil anschließend mit einer Hauptwaschmittelzusammensetzung gewaschen und anschließend vorzugsweise gespült und getrocknet wird.

13. Verwendung einer Lipase aus Moritella viscosa, die eine Sequenzidentität von mindestens 70% mit der SEQ.ID.1 aufweist, um eine verbesserte Reinigungswirkung bei Fettflecken, vorzugsweise Margarineflecken, auf Textilien zu erzielen.

14. Verwendung einer Reinigungsmittelzusammensetzung, die eine Lipase aus Moritella viscosa mit einer Sequenzidentität von mindestens 70% mit der SEQ.ID.1 umfasst, um eine verbesserte Reinigungswirkung bei Fettflecken, vorzugsweise Margarineflecken, auf Textilien zu erzielen.

15. Verwendung nach Anspruch 13 oder Anspruch 14, wobei die Lipase aus Moritella viscosa eine Sequenzidentität von mindestens 75%, bevorzugter 80%, bevorzugter 85%, noch bevorzugter 90%, noch bevorzugter 95%, noch bevorzugter 98%, noch bevorzugter 99% und höchst bevorzugt 100% mit der SEQ.ID.1 aufweist.

## Revendications

1. Composition détergente comprenant :
(a) de 0,0005 à 6 % en poids, de préférence de 0,005 à 4 % en poids, plus préférablement de 0,001 à 2 % en poids d'une lipase provenant de *Moritella viscosa*; et,
(b) de 1 à 60 % en poids, de préférence de 1 à 50 % en poids, plus préférablement de 1 à 35 % en poids d'un tensioactif détergent, dans laquelle la lipase provenant de *Moritella viscosa* présente une identité de séquence d'au moins 70 % avec la SEQ. ID. 1.

2. Composition détergente selon la revendication 1, dans laquelle la lipase provenant de *Moritella viscosa* présente une identité de séquence d'au moins 75 %, plus préférablement 80 %, plus préférablement 85 %, encore plus préférablement 90 %, encore plus préférablement 95 %, encore plus préférablement 98 %, encore plus préférablement 99 %, très préférablement 100 % avec la SEQ. ID. 1.

3. Composition détergente selon la revendication 1 ou la revendication 2, dans laquelle la composition détergente comprend un tensioactif anionique et/ou non-ionique, de préférence comprenant à la fois un tensioactif anionique et un tensioactif non-ionique.

4. Composition détergente selon la revendication 3, dans laquelle le tensioactif anionique est présent à un niveau de 1 à 50 % en poids, de préférence de 2 à 40 % en poids, plus préférablement de 3 à 30 % en poids et est de préférence choisi parmi un alkylbenzènesulfonate linéaire, un alcanesulfonate secondaire, le laureth-éthersulfate de sodium, le laurylsulfate de sodium, l'oléylsulfate de sodium et l'oléyl-éthersulfate de sodium, un méthyl-estersulfonate, un alkylsulfate secondaire (SALS), l'éther-sulfate de cardanol et un rhamnolipide.

5. Composition détergente selon la revendication 3 ou la revendication 4, dans laquelle le tensioactif non-ionique est présent à un niveau de 1 à 30 % en poids, de préférence de 2 à 20 % en poids, plus préférablement de 3 à 15 % en poids et est de préférence choisi parmi un alcool éthoxylé, un alcool propoxylé, un ester méthylique éthoxylé et un alkylpolyglycoside.

6. Composition détergente selon l'une quelconque des revendications précédentes, dans laquelle la composition détergente est une composition détergente de lessive.

7. Composition détergente de lessive selon la revendication 6, dans laquelle la composition détergente de lessive se présente sous la forme d'un liquide, d'un solide, d'une poudre, d'une pastille, d'une perle ou d'une pâte, de préférence la composition est un liquide ou une poudre, plus préférablement un détergent liquide.

8. Composition détergente de lessive selon la revendication 6 ou la revendication 7, dans laquelle la composition détergente de lessive comprend une polyamine alcoxylée, de préférence à un niveau de 0,1 à 8 % en poids, plus préférablement de 0,2 à 6 % en poids, très préférablement de 0,5 à 5 % en poids.

9. Composition détergente de lessive selon l'une quelconque des revendications 6 à 8, dans laquelle la composition détergente de lessive comprend un polymère de libération des salissures, le polymère de libération des salissures étant de préférence choisi parmi les copolyesters d'acides dicarboxyliques et de polydiols, plus préférablement un copolyester formé par condensation d'un ester d'acide téréphtalique et du 1,2-propanediol, le polymère de libération des salissures étant de préférence présent à un niveau de 0,1 à 8 % en poids, plus préférablement de 0,2 à 6 % en poids, très préférablement de 0,5 à 5 % en poids.

10. Composition détergente selon l'une quelconque des revendications précédentes, comprenant en outre une ou plusieurs enzymes supplémentaires choisies dans le groupe constitué des : protéases, cellulases, alpha-amylases, peroxydases/oxydases, pectate lyases et/ou mannanases, de préférence la ou les enzymes comprennent une protéase.

11. Procédé de traitement d'un textile présentant une tache grasse, dans lequel ledit textile est traité avec une composition selon l'une quelconque des revendications 1 à 10, afin de fournir un nettoyage lipolytique amélioré contre les taches grasses, de préférence les taches de margarine, ledit textile étant de préférence ensuite rincé et séché.

12. Procédé de traitement d'un textile présentant une tache grasse, dans lequel ledit textile est prétraité avec une composition selon la revendication 1 ou la revendication 2, afin de fournir un nettoyage lipolytique amélioré contre les taches grasses, de préférence les taches de margarine, ledit textile étant ensuite lavé avec une composition de lavage principal pour lessive, puis de préférence ensuite rincé et séché.

13. Utilisation d'une lipase provenant de *Moritella viscosa* présentant une identité de séquence d'au moins 70 % avec la SEQ. ID. 1, pour améliorer le nettoyage des taches grasses, de préférence des taches de margarine, sur les textiles.

14. Utilisation d'une composition détergente comprenant une lipase provenant de *Moritella viscosa* présentant une identité de séquence d'au moins 70 % avec la SEQ. ID. 1, pour améliorer le nettoyage des taches grasses, de préférence des taches de margarine, sur les textiles.

15. Utilisation selon la revendication 13 ou la revendication 14, dans laquelle la lipase provenant de *Moritella viscosa* présente une identité de séquence d'au moins 75 %, plus préférablement 80 %, plus préférablement 85 %, encore plus préférablement 90 %, encore plus préférablement 95 %, encore plus préférablement 98 %, encore plus préférablement 99 %, très préférablement 100 % avec la SEQ. ID. 1.
